# EUROPÄISCHE PATENTSCHRIFT

(11) **EP 2 273 966 B1**
(45) Veröffentlichungstag und Bekanntmachung des Hinweises auf die Patenterteilung: **21.03.2018**
(21) Anmeldenummer: 09745639.6
(22) Anmeldetag: 15.04.2009
(51) Int. Cl.: A61K 8/06, A61K 8/89, A61Q 19/00, A61Q 1/02, A61Q 17/04, A61Q 19/04, A61Q 19/08, C11D 3/37, C08G 77/04, C08G 77/20, C08G 77/44

(54) **VERWENDUNG ORGANOMODIFIZIERTER SILOXANBLOCKCOPOLYMERE ZUR HERSTELLUNG KOSMETISCHER ZUSAMMENSETZUNGEN**
USE OF ORGANO-MODIFIED SILOXANE BLOCK COPOLYMERS FOR PRODUCING COSMETIC COMPOSITIONS
UTILISATION DE COPOLYMÈRES BLOCS DE SILOXANE ORGANOMODIFIÉS POUR PRODUIRE DES COMPOSITIONS COSMÉTIQUES

(30) Priorität: 15.05.2008 DE 102008001788
(43) Veröffentlichungstag der Anmeldung: 19.01.2011
(73) Patentinhaber: Evonik Degussa GmbH, 45128 Essen (DE)
(72) Erfinder: MEYER, Jürgen, 45134 Essen (DE); UNGER, Frank, 47167 Duisburg (DE); FERENZ, Michael, 45147 Essen (DE); HERRWERTH, Sascha, 45134 Essen (DE); HARTUNG, Christian, 45133 Essen (DE); LOHSE, Andrea, 46236 Bottrop (DE)
(86) Internationale Anmeldenummer: PCT/EP2009/054426
(87) Internationale Veröffentlichungsnummer: WO 2009/138306

(56) Entgegenhaltungen:
- EP-A- 0 298 402
- EP-A- 1 125 574
- EP-A- 1 679 335
- EP-A- 1 892 327
- DE-A1-102005 001 040

## Beschreibung

### Gebiet der Erfindung:

Die Erfindung betrifft Emulgator-Systeme, die organomodifizierte Siloxanblockcopolymere umfassen, sowie kosmetische Formulierungen, die diese Emulgator-Systeme enthalten.

### Stand der Technik:

Organomodifizierte Siloxane werden in den verschiedensten Applikationen eingesetzt. Ihre Eigenschaften lassen sich unter anderem durch die Art der Modifikation, sowie durch die Modifikationsdichte gezielt einstellen.
So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren, als Entschäumer in Treibstoffen oder als Additive in Farben und Lacken.
So beschreibt z.B. DE 102005001041 funktionalisierte Polyorganosiloxane und deren Einsatz als Kraftstoffentschäumer. Die Allylpolyether in den hier dargestellten Siloxanen können gegebenenfalls durch Abänderung der Synthese durch Kohlenwasserstoffreste ersetzt werden.

Generell können Siloxane durch Umsetzung mit z.B. α-Olefinen mit hydrophoben Gruppen verknüpft werden. Die so erhaltenen Siliconwachse dienen zum Beispiel als Additiv in Personal-Care-Applikationen.

Es zeigt sich in vielen Anwendungsgebieten, dass die Wirkung des Siloxans entscheidend von der Verträglichkeit mit der entsprechenden Formulierung abhängt.

Als kosmetischer Emulgator geeignet sind zum Beispiel Siloxane, die neben aliphatischen Gruppen auf Basis von α-Olefinen Polyether tragen. Als typisches Beispiel ist hier das Verkaufsprodukt ABIL EM 90 der Evonik Goldschmidt GmbH (Deutschland) zu nennen, das sich insbesondere durch eine hervorragende Stabilisierung von Wasser-in-Öl (W/O) Emulsionen auszeichnet.

Siloxanbasierte Emulgatoren für Öl-in-Wasser (O/W) Emulsionen müssen einen hydrophileren Charakter aufweisen, weshalb es sich bei diesen Produkten in der Regel um reine Polyethersiloxane handelt.

EP 1125574 beschreibt die Verwendung relativ hydrophober Polyethersiloxane als O/W-Emulgatoren, bei denen sich die Polyethergruppen α-ω- oder endständig am Siloxanrücken befinden. Diese Strukturen zeichnen sich insbesondere durch ein samtig seidiges Hautgefühl aus, das sie in kosmetische Emulsionen einzubringen vermögen.

Nachteilig an der Verwendung dieser Strukturen ist die oft nicht ausreichende Emulgierfähigkeit.

EP0298402 beschreibt die Verwendung von Organopolysiloxan-Polyoxyalkylenen als Emulgator in Wasser-in-Öl-Emulsionen und stellt den bekannten, nächsten Stand der Technik dar. Die Organopolysiloxan-Polyoxyalkylene zeichnen sich dadurch aus, dass zwei Organopolysiloxan-Polyoxyalkylen-Moleküle über einen möglichst kurzen, nicht hydrolisierbaren Crosslinker miteinander verbunden sind. Während der Herstellung dieser Polymere ist das molare Verhältnis von vinylischem Crosslinker zu zu verknüpfenden Organopolysiloxan-Polyoxyalkylen-Molekülen sehr groß. Dies führt zu unvorteilhaften Nebenprodukten. Unter anderem auch aus diesem Grund sind die in der EP0298402 beschriebenen Emulgator-Systeme für kosmetische Anwendungen nur mit Einschränkungen geeignet.
Eine Aufgabe der vorliegenden Erfindung bestand daher darin, neuartige, organomodifizierte Siloxane zu entwickeln, die als leistungsfähige Emulgatoren sowohl in O/W als auch in W/O Emulsionen eingesetzt werden können und dabei insbesondere in O/W Emulsionen in der Lage sind, ein samtig-seidiges Hautgefühl mit herausragenden Emulgiereigenschaften zu kombinieren.
Darüber hinaus sollten diese Siloxane vorzugsweise einfach zu verarbeiten sein (flüssig bei Raumtemperatur) und mit herkömmlichen Siloxanen in Formulierungen kombiniert werden können.

### Beschreibung der Erfindung:

Überraschenderweise wurde gefunden, dass organomodifizierte Siloxanblockcopolymere, definiert in Anspruch 1, leistungsfähige Emulgatoren, besonders für kosmetische Formulierungen, darstellen, die diese Aufgabe zu lösen vermögen.

Gegenstand der Erfindung ist daher die Verwendung eines Emulgator-Systems die organomodifizierte Siloxanblockcopolymere, definiert in Anspruch 1, enthalten.
Ein besonderer Vorteil der erfindungsgemäßen Emulgator-Systeme sind deren hervorragenden emulgierenden und stabilisierenden Eigenschaften.
Ein weiterer Vorteil ist, dass sie es ermöglichen, in kosmetischen Formulierungen ein samtig-seidiges Hautgefühl einzubringen. Dies ist insofern wichtig, da Konsumenten zunehmend Wert darauf legen, dass kosmetische Formulierungen sich nicht nur leicht verteilen lassen und gut in die Haut einziehen, sondern dass nach dem Einziehen ein glatter, weicher, samtiger Eindruck verbleibt.

Ein weiterer Vorteil der Emulgator-Systeme ist, dass die Eigenschaften von seitenständig modifizierten Siloxanen und von α,ω-modifizierten Siloxanen in ihnen vereint vorliegen, und somit ein höherer Modifikationsgrad im Sinne einer größeren Anzahl an Substitutionsmöglichkeiten gegeben ist.

Die Emulgator-Systeme werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z.B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen.

Gegenstand der Erfindung ist daher die Verwendung mindestens eines Emulgator-Systeme, enthaltend organomodifizierte Siloxanblockcopolymere, erhältlich durch
A) Anlagerung von Organopolysiloxanen der allgemeinen Formel I 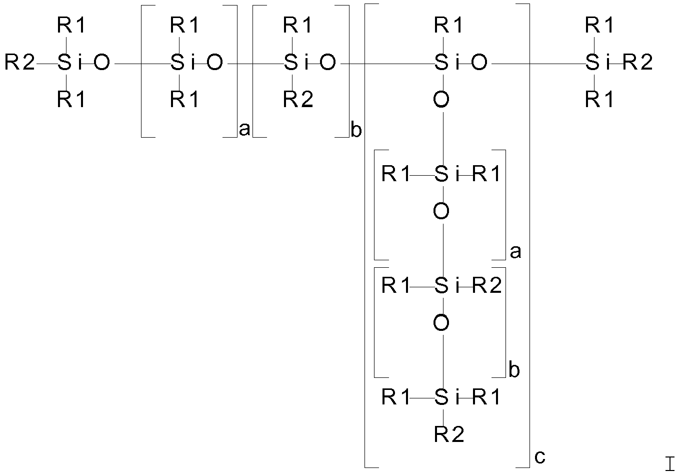 worin
   - R¹: gleiche oder verschiedene, verzweigte oder unverzweigte, aliphatische oder aromatische Kohlenwasserstoffreste mit 1 bis 20 C-Atomen,
   - R²: R¹ oder H, mit der Maßgabe, daß mindestens drei Reste R² gleich H sind,
   - a: 5 bis 500, bevorzugt 10 bis 250, insbesondere 15 bis 75, b 1 bis 50, bevorzugt 1 bis 20, insbesondere 3 bis 15,
   - c: 0 bis 5, bevorzugt 0 bis 1, insbesondere 0,
   bedeuten,
   an Doppelbindungen enthaltende Siloxane der allgemeinen Formel II, 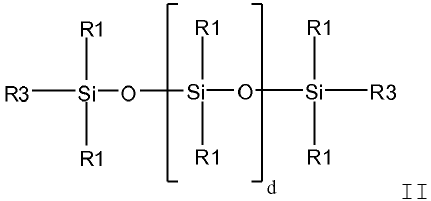 mit
   d 10 bis 1.000, bevorzugt 101 bis 750, insbesondere 201 bis 500 und
   R³ unabhängig voneinander gleiche oder verschiedene, mindestens eine Doppelbindung enthaltende Kohlenwasserstoffreste mit 2 bis 12, bevorzugt 2 bis 8, insbesondere 2 C-Atomen,
   in Gegenwart von Platin- oder Rhodiumkatalysatoren, mit der Maßgabe, dass die Organopolysiloxane der allgemeinen Formel I im mindestens 6-fachen molaren Überschuss, bezogen auf das Doppelbindungen enthaltende Siloxan der allgemeinen Formel II, vorliegt, unter Erhalt eines Si-H-Gruppen aufweisenden Reaktionsproduktes und weiterer Umsetzung des Reaktionsproduktes in mindestens einer der Stufen
B) übergangsmetall-katalysierter teilweiser oder vollständiger Addition der SiH-Gruppen an Alkenyl- und/oder Alkinylverbindungen, bevorzugt an Doppelbindungen enthaltende Polyether und α-Olefine, insbesondere an Allylpolyether,
   oder
C) teilweise oder vollständige Umsetzung der nach obiger Reaktion(en) verbliebenen Si-H-Gruppen in Gegenwart eines Katalysators mit mindestens einem Alkohol, aus der Gruppe der linearen oder verzweigten, gesättigten, ein- oder mehrfach ungesättigten, aromatischen, aliphatisch-aromatischen, gegebenenfalls Halogenatome enthaltenden Monoalkohole, Polyethermonoalkohole, Polyestermonoalkohole, Aminoalkohole.

Die Reste R¹ sind bevorzugt gleiche oder verschiedene aliphatische oder aromatische Kohlenwasserstoffreste mit 1 bis 20 C-Atomen, weiter bevorzugt gleiche oder verschiedene unverzweigte, aliphatische oder aromatische Kohlenwasserstoffreste mit 1 bis 9 C-Atomen und besonders bevorzugt Methyl, Ethyl oder Phenyl.

Durch die Maßgabe, dass das SiH-Gruppen tragende Organopolysiloxan der allgemeinen Formel I im mindestens 6-fachen molaren Überschuss, bezogen auf das Doppelbindungen enthaltende Siloxan der allgemeinen Formel II, vorliegt, wird verhindert, dass es zur Ausbildung eines Netzwerkes und zur Entstehung hochviskoser Produkte kommt. In der Regel besitzen die nach einem der beiden vorgenannten Verfahren hergestellten Organosiloxane Viskositäten bis 10.000 mPas. Ein gewisser Anteil des Organosiloxan kann in Form eines kammartig modifizierten Siloxans im Produkt vorliegen.

Aufgrund der gewählten Reaktionsbedingungen bildet sich aus dem Doppelbindungen enthaltende Siloxan und dem Sifunktionellen Siloxan in der ersten Stufe ein Siloxan der folgenden in Formel III dargestellten idealisierten "H-Struktur" (c = 0, R¹ = Me, R² = R = Me oder H): 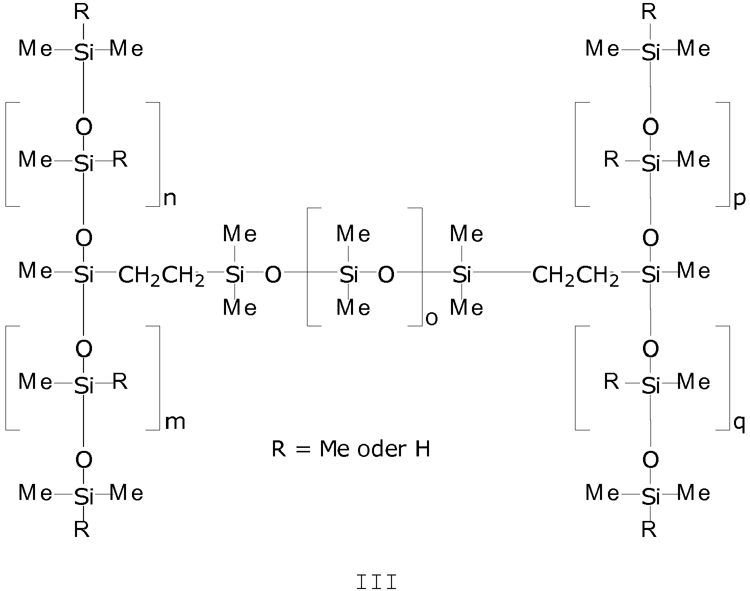 wobei m, n, o, p und q positive, ganze Zahlen sind.

Dieses Siloxangerüst bleibt bei den anschließenden Reaktionsstufen erhalten. Die Darstellung der Siloxanpolymere kann mit Lösemittel oder lösemittelfrei erfolgen. Die gegebenenfalls aufkommende Schaumbildung kann durch den Einsatz von Lösemitteln unterdrückt werden.

Geeignete Lösemittel sind zum Beispiel Toluol und Cyclohexan.

Als wirksame Katalysatoren für den ersten Schritt, der Hydrosilylierung des Doppelbindungen enthaltende Siloxans A), können Pt- und Rh-haltige Komplexe eingesetzt werden, die dem Fachmann als hydrosilylierungsaktive Katalysatoren bekannt sind, zum Beispiel: H₂PtCl₆, Pt [(CH₂=CH-SiMe₂)₂O]ₙ oder Rh (CO) (C₅H₇O₂).

Für die Anlagerung des Alkohols in Verfahrensschritt C) an das erhaltene SiH-haltige Siloxan können zum Beispiel Lewissäuren, bevorzugt Bor-haltige Lewissäuren, eingesetzt werden. Als borhaltige Verbindungen des katalytischen Systems können fluorierte und/oder nicht-fluorierte Organoborverbindungen eingesetzt werden, insbesondere solche, die ausgewählt sind aus:
(C₅F₄)(C₆F₅)₂B; (C₅F₄)₃B; (C₆F₅)BF₂; BF(C₆F₅)₂; B(C₆F₅)₃; BCl₂(C₆F₅); BCl(C₆F₅)₂; B(C₆H₅) (C₆F₅)_{2;} B(C₆H₅)₂(C₆F₅); [C₆H₄ (m-CF₃)]₃B; [C₆H₄(p-OCF₃)]₃B; (C₆F₅) B (OH) ₂; (C₆F₅)₂BOH; (C₆F₅)₂BH; (C₆F₅)BH₂; (C₇H₁₁)B (C₆F₅)₂; (C₈H₁₄B) (C₆F₅) ; (C₆F₅)₂B(OC₂H₅) ; (C₆F₅)₂B-CH₂CH₂Si(CH₃)₃; 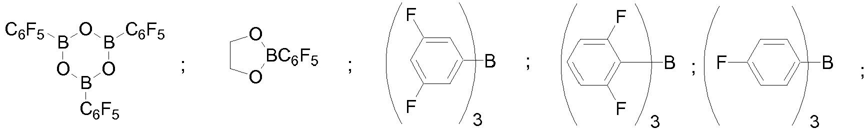 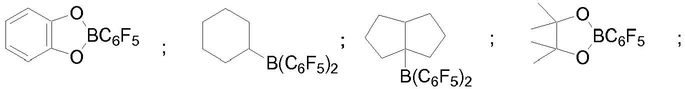 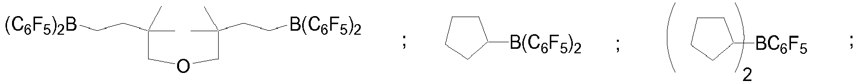 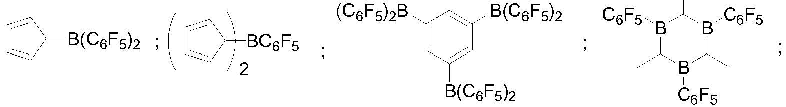 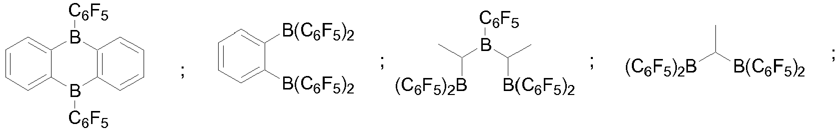
besonders bevorzugt Tris(pentafluorphenyl)boran [CAS-Nr. 1109-15-5], sowie Gemische der vorstehenden Katalysatoren. Beim Einsatz dieser borhaltigen Katalysatoren können des Weiteren synergistisch wirksame Verbindungen eingesetzt werden. Dazu zählen Salze oder Komplexe, mit Kationen ausgewählt aus der Gruppe der Salze von Elementen der 4., 6., 7. und 8. Nebengruppe sowie der 4. Hauptgruppe. Als Anionen der synergistisch aktiven Verbindungen des katalytischen Systems können bevorzugt Alkoxylate, Säureanionen, insbesondere Carboxylate, Sulfate, Nitrate oder Phosphate, Halogenide, insbesondere Chloride, Oxide oder Komplexliganden, insbesondere Acetylacetonat oder Carbonyle, verwendet werden.

Ferner wird in DE10312634.1 ein Verfahren zur Herstellung organisch modifizierter Polyorganosiloxane unter Verwendung einer katalytischen Mischung enthaltend mindestens eine Carbonsäure und mindestens ein Salz einer Carbonsäure durch Verknüpfung von Wasserstoffsiloxanen mit Alkoholen beschrieben. Auch diese Katalysatoren können in Verfahrensschritt C) eingesetzt werden.

Geeignete Alkohole sind zum Beispiel lineare oder verzweigte, gesättigte, ein- oder mehrfach ungesättigte, aromatische, aliphatisch-aromatische Mono- oder Polyalkohole, Polyethermonoalkohole, Polyetherpolyalkohole, Polyestermonoalkohole, Polyesterpolyalkohole, Aminoalkohole, insbesondere N-Alkyl-, Arylamino-EO-, -PO-Alkohole (EO steht für den Polyethylenoxidrest, PO für den Polypropylenoxidrest), N-Alkyl- oder Arylaminoalkohole sowie deren Gemische. Besonders geeignet sind Polyethermonoalkohole.

Als wirksame Katalysatoren für die Übergansmetallkatalysierte Addition der SiH-Gruppen des im ersten Schritt hergestellten Siloxans an C C-Mehrfachbindungen in Verfahrenschritt B) können die bekannten Hydrosilylierungskatalysatoren verwendet werden, zum Beispiel: H₂PtCl₆, Pt [(CH₂=CH-SiMe₂)₂O]ₙ oder Rh (CO)(C₅H₇O₂).

Geeignete Alkenyl-/Alkinylverbindungen sind zum Beispiel Polyether mit Mehrfachbindungen, zum Beispiel Butindiolalkoxylate oder allylfunktionelle Polyether, Olefine, Ethen, Ethin, Propen, 1-Buten, 1-Hexen, 1-Dodecen, 1-Hexadecen, Allylalkohol, Hex-5-en-1-ol, Styrol, Eugenol, Allylphenol, Undecylensäuremethylester. Besonders geeignet sind Polyether mit Doppenbindungen insbesondere allylfunktionelle Polyether.

Die erfindungsgemäßen Emulgator-Systeme werden vorzugsweise als Öl-in-Wasser-, Wasser-in-Öl- oder Wasser-in-Silicon-Emulgatoren oder Dispergierhilfsmittel verwendet. Gegenstand der vorliegenden Erfindung sind deshalb auch kosmetische Formulierung oder Pflege- und Reinigungsmittel enthaltend mindestens eines der erfindungsgemäßen Emulgator-Systeme. Das Emulgator-System könnte auch zur Herstellung von O/W-Tränkemulsionen für Textilien verwendet. Bevorzugt handelt es sich bei den Textilien um Feuchttücher, besonders bevorzugt um kosmetische Feuchttücher.

Die Emulsionen und Dispersionen enthalten bezogen auf die Gesamtmasse mehr Massenprozent Ölkomponente als die Summe der Massenprozente von Emulgator, Tensid und ggfs. Co-Emulgator.
Somit sind die mit Hilfe der erfindungsgemäßen Emulgator-Systeme erhaltenen Öl-in-Wasser-, Wasser-in-Öl- und Wasser-in-Silicon-Emulsionen und Dispersionen, sowie O/W-Tränkemulsionen für Textilien, ebenfalls Gegenstand der Erfindung. Ebenso sind die mit erfindungsgemäßen O/W-Tränkemulsionen getränkten Textilien Gegenstand der Erfindung. Diese zeichnen sich durch eine gute Reinigungsleistung und ein angenehm samtig-glattes Hautgefühl aus.

Die kosmetischen Formulierungen sowie die Pflege- und Reinigungsmittel können z.B. mindestens eine zusätzliche Komponenten enthalten, ausgewählt aus der Gruppe der
Emollients,
Emulgatoren und Tenside,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Kosmetische Wirkstoffe,
Pflegeadditive,
Überfettungsmittel,
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butylsebacat, Di-(2-ethylhexyl)-adipat, Di-(2-hexyldecyl)-succinat, Di-isotridecylacelaat. Geeignete Diolester sind z.B. Ethylenglycoldioleat, Ethylenglycol-di-isotridecanoat, Propylenglycol-di-(2-ethylhexanoat), Butandiol-diisostearat, Butandiol-di-caprylat/caprat und Neopentylglycol-di-caprylat. Weitere Fettsäureester, die als Emollients eingesetzt werden können, sind z.B. C₁₂₋₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆- C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten C₈-C₁₈-Alkoholen, insbesondere 2-Ethylhexanol oder Isononanol, Ester von verzweigten C₆-C₁₃-Carbonsäuren mit verzweigten Alkoholen, insbesondere 2-Ethylhexanol oder Isononanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C6-C18-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv™ TN), Di alkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden.

Als nichtionogene Emulgatoren oder Tenside können Verbindungen aus mindestens einer der folgenden Gruppen eingesetzt werden:
Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C_{12/18}-Fettsäuremono- und -diester von Anlagerungsprodukten von 1 bis 100 Mol Ethylenoxid an Glycerin,
Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte,
Anlagerungsprodukte von 2 bis 200 Mol Ethylenoxid an Ricinusöl und/oder gehärtetes Ricinusöl,
Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-C opolymere bzw. entsprechende Derivate, wie z.B. Lauryl oder Cetyl Dimethicone Copolyole, insbesondere Cetyl PEG/PPG-10/1 Dimethicone (ABIL® EM 90 (Evonik)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin, Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid.
Weiterhin können Monoalklyamidoquats wie z.B. Palmitamidopropyltrimethylammoniumchlorid oder entsprechende Dialkylamidoquats eingesetzt werden. Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.
Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen. Weiterhin ist es möglich, amphotere Tenside wie z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.
Von den ampholytischen Tensiden können solche oberflächenaktiven Verbindungen eingesetzt werden, die außer einer C8/18-Alkyl- oder -Acylgruppe im Molekül mindestens eine freie Aminogruppe und mindestens eine - COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C12/18-Acylsarcosin.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole TM oder Synthalene TM), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z.B. Zinkstearat eingesetzt werden.

Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:
3-Benzylidencampher und dessen Derivate, z.B. 3-(4-Methylbenzyliden)campher, 4-Aminobenzoesäurederivate, wie z.B. 4-(Dimethylamino)benzoesäure-2-ethylhexylester und 4-(Dimethylamino)benzoesäureamylester
Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene),
Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon, Ester der Benzalmalonsäure, wie z.B. 4-Methoxybenzmalonsäuredi-2-ethylhexyester,
Triazinderivate, wie z.B. 2,4,6-Trianilino-(p-carbo-2'-ethyl-1'-hexyloxy)-1,3,5-triazin und Octyltriazon, Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage:
2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze,
Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen. Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Als Antioxidantien können z.B. Superoxid-Dismutase, Tocopherole (Vitamin E), Dibutylhydroxytoluol und Ascorbinsäure (Vitamin C) eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filter- noch färbende Wirkung haben, beispielsweise Aerosile® (CAS-Nr. 7631-86-9).

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Illit, Beidelit, Nontronit, Saponit, Hectorit, Bentonit, Smectit, ferner beispielsweise Zinksalze der Ricinolsäure in Frage. Keimhemmende Mittel sind ebenfalls geeignet, eingearbeitet zu werden. Keimhemmende Substanzen sind zum Beispiel 2,4,4'-Trichlor-2'-hydroxydiphenylether (Irgasan), 1,6-Di-(4-chlorphenylbiguanido) -hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Evonik), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salizylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethyl-isothiazolin, Ethylhexylglycerin oder Caprylyl Glycol eingesetzt werden. Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter kosmetischen Wirkstoffen sind beispielsweise Tocopherol, Tocopherolacetat, Tocopherolpalmitat, Ascorbinsäure, Desoxyribonucleinsäure, Coenzym Q10, Retinol, Bisabolol, Allantoin, Phytantriol, Panthenol, AHA-Säuren, Aminosäuren, Hyaluronsäure, alpha-Hydroxysäuren, Polyglutaminsäure, Creatin (und Creatinderivate), Guanidin (und Guanidinderivate), Ceramide, Phytosphingosin (und Phytosphingosinderivate), Sphingosin (und Sphingosinderivate), Pseudoceramide, Sphingolipide, essentielle Öle, Peptide und Oligopeptide, Proteinhydrolysate, Pflanzenextrakte und Vitaminkomplexe zu verstehen.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.
In einer bevorzugten Ausführungsform enthalten die kosmetischen Formulierungen sowie die Pflege- und Reinigungsmittel als zusätzliche Komponente Pigmente (z.B. TiO₂, FeOₓ, ZnO, Mica und beispielsweise solche, die unter UV-Filtersubstanzen und Feststoffe gelistet) oder Partikel (z.B. Siliconelastomere, Nylon-12, PMMA, Bornitrid und beispielsweise solche, die unter UV-Filtersubstanzen und Feststoffe gelistet).
In einer ebenso bevorzugten Ausführungsform enthalten die kosmetischen Formulierungen sowie die Pflege- und Reinigungsmittel als zusätzliche Komponente kosmetische Wirkstoffe.

Als Applikationsformen der Emulsionen und Dispersionen enthaltend das Emulgator-System sind daher Sprays, Lotionen, Cremes, Salben und somit der Einsatz über einen sehr breiten Konsistenzbereich von wasserdünn bis stark pastös, im Extremfall sogar fest, möglich.
Daher können die Emulgator-Systeme beispielsweise in Pflegecremes und -lotionen für Gesicht, Körper und Hände, in Sonnenschutzemulsionen, in Schminken, in Aersolen, Rollons, Pumpsprays, Stiften z.B. im AP/Deo-Bereich, in Babypflegeprodukten, in Intimpflege-, Fußpflege-, Haarpflege-, Nagelpflege, Zahnpflege- oder Mundpflegeprodukten sowie in dermatologischen Salben eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Emulgatorbeispiele:

### Emulgator 1 (erfindungsgemäß) (O/W Emulgator):

Herstellung: Zunächst wurden 183 g eines SiH-funktionellen Siloxans der allgemeinen Formel Me₃SiO(SiMe₂O)₂₈(SiMeHO)₁₀SiMe₃ mit 143 g eines Vinylsiloxans der allgemeinen Formel CH₂=CH-SiMe₂O- (SiMe₂O)₃₄₈-SiMe₂-CH=CH₂ gemischt, auf 120 °C geheizt und mit 1 ppm eines Rh-Katalysators versetzt. Nach 30 min wurden dann 1322 g eines Allylpolyethers der allgemeinen Formel CH₂=CH-CH₂-O-(CH₂CH₂O)₂₅ (CH₂CH (CH₃) O)₄Me zugesetzt auf 94 °C abkühlen gelassen und 8 ppm eines Platin-Katalysators zugesetzt. Anschließend wurde noch 2 h bei 120 °C weitergerührt.

### Emulgator 2 (erfindungsgemäß) (O/W Emulgator):

Herstellung: Zunächst wurden 42 g eines SiH-funktionellen Siloxans der allgemeinen Formel Me₃SiO(SiMe₂O)₃₈-(SiMeHO)₉SiMe₃ mit 21 g eines Vinylsiloxans der allgemeinen Formel CH₂=CH-SiMe₂O-(SiMe₂O)₂₈₀-SiMe₂-CH=CH₂ gemischt, auf 120 °C geheizt und mit 1 ppm eines Rh-Katalysators versetzt. Nach 30 min wurden dann 148 g eines Allylpolyethers der allgemeinen Formel CH₂=CH-CH₂-O-(CH₂CH₂O)₂₅(CH₂CH(CH₃)O)₄Me zugesetzt auf 90 °C abkühlen gelassen und 10 ppm eines Pt-Katalysators zugesetzt. Anschließend wurde noch 2 h bei 120 °C weitergerührt und das Produkt filtriert.

### Emulgator 3 (erfindungsgemäß) (W/O Emulgator)

Herstellung: Zunächst wurden 144 g eines SiH-funktionellen Siloxans der allgemeinen Formel Me₃SiO(SiMe₂O)₇₃(SiMeHO)₂₅SiMe₃ mit 26 g eines Vinylsiloxans der allgemeinen Formel CH₂=CH-SiMe₂O-(SiMe₂O)₃₄₈-SiMe₂-CH=CH₂ gemischt, auf 90 °C geheizt und mit 5 ppm eines Platin-Katalysators versetzt. Anschließend wurden dann 32 g eines Allylpolyethers der allgemeinen Formel CH₂=CH-CH₂-O-(CH₂CH₂O)₈OH sowie 117 g 1-Hexadecen zugesetzt. Dabei stieg die Temperatur bis auf 113 °C. Anschließend wurde noch 2 h bei 110 °C weitergerührt und das Produkt im Vakuum von flüchtigen Bestandteilen befreit.

### Vergleichsbeispiele 1-6 (nicht erfindungsgemäß, zur Abgrenzung vom Stand der Technik):

Die Struktur der Vergleichsemulgatoren 1 bis 5 entspricht der allgemeinen Formel:

R₁(CH₃)₂SiO-[(CH₃)₂SiO]ₙ-[(CH₃) R₂SiO]ₘ-Si(CH₃)₂R₁,

Mit:R₁, R₂ = CH₃ oder ein Polyether ("PE") des Typs: -(CH₂)_{w}-O-(C₂H₄O)ₓ-C₃H₆O)y-R₃ mit R₃ = H oder CH₃

| Vergl. Emulg. | **n** | **m** | **R₁** | **R₂** | **R₃** | **w** | **x** | **y** |
|---|---|---|---|---|---|---|---|---|
| 1 | 66 | 0 | PE | - | H | 3 | 13 | 0 |
| 2 | 50 | 0 | PE | - | CH₃ | 3 | 15 | 10 |
| 3 | 200 | 0 | PE | - | H | 3 | 13 | 20 |
| 4 | 100 | 0 | PE | - | H | 3 | 11 | 17 |
| 5 | 45 | 5 | CH₃ | PE | H | 3 | 20 | 20 |

Die Vergleichsemulgatoren 1 - 4 entsprechen den Beispielen 1-4 der EP 1125574.

Bei Vergleichsemulgator 5 handelt es sich um einen typischen kammartig aufgebauten Siliconpolyether.

Ein weiterer Vergleichsemulgator 6 unterscheidet sich von erfindungsgemäßen Emulgatoren durch die sehr kurze Siloxankettenlänge des verbrückenden Siloxans, die mit d = 0 außerhalb des Anspruchsbereichs dieser Erfindung liegt.
Herstellung Vergleichsemulgator 6 (nicht erfindungsgemäß) (O/W Emulgator):
Zunächst wurden 240 g eines SiH-funktionellen Siloxans der allgemeinen Formel Me₃SiO(SiMe₂O)₂₈(SiMeHO)₁₀SiMe₃ mit 1,3 g eines Vinylsiloxans der allgemeinen Formel CH₂=CH-SiMe₂O-SiMe₂-CH=CH₂ gemischt, auf 120 °C geheizt und das Gemisch mit 1 ppm eines Rh-Katalysators versetzt. Nach 30 min wurden dann 1732 g eines Allylpolyethers der allgemeinen Formel CH₂=CH-CH₂-O-(CH₂CH₂O)₂₅(CH₂CH(CH₃)O)₄Me zugesetzt auf 94 °C abkühlen gelassen und 8 ppm eines Platin-Katalysators zugesetzt. Anschließend wurde noch 2 h bei 120 °C weitergerührt.

### Anwendungsbeispiele:

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

### Emulgierleistung

Zur Überprüfung der Emulgierleistung in O/W Emulsionen wurde ein Schnelltest eingesetzt, der unter sehr kritischen Bedingungen (nur 0,5 % Emulgator) sehr schnell zeigt, welche Strukturen sich durch hervorragende Emulgieraktivität auszeichnen.

Unter Verwendung üblicher Öle und Stabilisatoren zeigt insbesondere die Stabilität nach 24 h Lagerung bei 50°C sehr deutlich, ob ein Emulgator sehr gute stabilisierende Eigenschaften hat.

Die Ergebnisse der erfindungsgemäßen Emulgatoren 1 und 2 sind im Vergleich zu den Ergebnissen der Vergleichsemulgatoren 1 bis 5 in Tabelle 1 zusammengefasst.

Die Herstellung der Emulsionen erfolgte dabei nach folgendem Verfahren:

Die Phasen A und B werden bei Raumtemperatur gemischt, Phase C wird ohne Rühren zugegeben. Anschließend wird 1 min homogenisiert. Die Phasen D und E werden zugegeben, anschließend wieder 1 min homogenisiert.

Die Ergebnisse der Emulsionsbeispiele 1 und 2 zeigen, dass die erfindungsgemäßen Emulgatoren deutlich höhere Stabilisierungseigenschaften besitzen als die Vergleichsemulgatoren 1 - 4 (α-ω modifizierte Siloxane aus EP 1125574) oder Vergleichsemulgator 5 (typisches kammartig modifiziertes Polyethersiloxan).

**Tabelle 1: Zusammensetzung und Bewertung der Untersuchungen im Emulsionsschnelltest.**

| | **Beispiele** | **1** | **2** | **V1** | **V2** | **V3** | **V4** | **V5** |
|---|---|---|---|---|---|---|---|---|
| A | Emulgator 1 | 0,5% | | | | | | |
| | Emulgator 2 | | 0,5% | | | | | |
| | Vergleichsemulgator 1 | | | 0,5% | | | | |
| | Vergleichsemulgator 2 | | | | 0,5% | | | |
| | Vergleichsemulgator 3 | | | | | 0,5% | | |
| | Vergleichsemulgator 4 | | | | | | 0,5% | |
| | Vergleichsemulgator 5 | | | | | | | 0,5% |
| | Ethylhexyl Stearate | 9,0% | 9,0% | 9,0% | 9,0% | 9,0% | 9,0% | 9,0% |
| | Paraffinum Perliquidum | 9,0% | 9,0% | 9,0% | 9,0% | 9,0% | 9,0% | 9,0% |
| | Ethanol | 5,0% | 5,0% | 5,0% | 5,0% | 5,0% | 5,0% | 5,0% |
| B | Carbomer | 0,16% | 0,16% | 0,16% | 0,16% | 0,16% | 0,16% | 0,16% |
| | Ethylhexyl Stearate | 1,04% | 1,04% | 1,04% | 1,04% | 1,04% | 1,04% | 1,04% |
| C | Demineralized Water | ad 100% | ad 100% | ad 100% | ad 100% | ad 100% | ad 100% | ad 100% |
| D | NaOH (5% solution) | 1,25% | 1,25% | 1,25% | 1,25% | 1,25% | 1,25% | 1,25% |
| E | Euxyl® K 300¹⁾ | 0,70% | 0,70% | 0,70% | 0,70% | 0,70% | 0,70% | 0,70% |
| | | | | | | | | |
| | Stabilität nach 24 h bei 50°C | stabil | stabil | Ölseparation starke Koaleszenz | Ölseparation starke Koaleszenz | Ölseparation starke Koaleszenz | Ölseparation starke Koaleszenz | Ölseparation starke Koaleszenz |

| | | | | | | | | |
|---|---|---|---|---|---|---|---|---|
| ¹⁾Euxyl® K 300(Schülke & Mayr): Phenoxyethanol, Methylparaben, Ethylparaben, Propylparaben, Butylparaben, Isopropylparaben | | | | | | | | |

### Hautgefühl und Emulsionsstabilität:

Um Hautgefühl und Stabilität der beiden erfindungsgemäßen Emulgatorbeispiele 1 und 2 zu untersuchen, wurden diese in einer Konzentration von 2% in einer kosmetischen Formulierung eingesetzt (Emulsionsbeispiele 3 und 4).

Als Vergleichsbeispiele dienten die Vergleichsemulgatoren 1, 2, 4, 5 und 6 (Vergleichsemulsionsbeispiele V6 - V10).

Das Hautgefühl der entsprechenden Emulsion wurde in einem Panel von 10 Personen im Vergleich jeweils zur Formulierung mit Vergleichsemulgator 1 bewertet.

Die Testergebnisse sind in Tabelle 2 zusammengefasst.

In diesen Beispielformulierungen wird deutlich, dass es lediglich mit den erfindungsgemäßen Emulgatoren möglich ist, sowohl stabile als auch vom Hautgefühl vorteilhafte Formulierungen herzustellen.

Insbesondere wird in V10 gezeigt, dass ein außerhalb von Anspruch 1 liegendes verbrücktes Siloxan zwar zu einer relativ guten Emulsionsstabilität führt, dass das Hautgefühl der Formulierung aber deutlich hinter den erfindungsgemäßen Beispielen zurück bleibt.

**Tabelle 2: Hautgefühl und Stabilitätsdaten von Testemulsionen**

| **Beispiele** | **3** | **4** | **V6** | **V7** | **V8** | **V9** | **V10** |
|---|---|---|---|---|---|---|---|
| Emulgator 1 | 2,00% | | | | | | |
| Emulgator 2 | | 2,00% | | | | | |
| Vergleichsemulgator 1 | | | 2,00% | | | | |
| Vergleichsemulgator 2 | | | | 2,00% | | | |
| Vergleichsemulgator 4 | | | | | 2,00% | | |
| Vergleichsemulgator 5 | | | | | | 2,00% | |
| Vergleichsemulgator 6 | | | | | | | 2,00% |
| Ethylhexyl Stearate | 10,0% | 10,0% | 10,0% | 10,0% | 10,0% | 10,0% | 10,0% |
| Paraffinum Perliquidum | 9,0% | 9,0% | 9,0% | 9,0% | 9,0% | 9,0% | 9,0% |
| Carbomer | 0,16% | 0,16% | 0,16% | 0,16% | 0,16% | 0,16% | 0,16% |
| Xanthan Gum | 0,16% | 0,16% | 0,16% | 0,16% | 0,16% | 0,16% | 0,16% |
| Demineralized Water | ad 100% | ad 100% | ad 100% | ad 100% | ad 100% | ad 100% | ad 100% |
| NaOH (5% solution) | 1,25% | 1,25% | 1,25% | 1,25% | 1,25% | 1,25% | 1,25% |
| Euxyl® K 300¹⁾ | 0,70% | 0,70% | 0,70% | 0,70% | 0,70% | 0,70% | 0,70% |
| | | | | | | | |
| Stabilität nach 3 Monaten | stabil | stabil | Ölseparation starke Koaleszenz | Ölseparation starke Koaleszenz | Ölseparation starke Koaleszenz | Starke Koaleszenz | leichte Koaleszenz |
| Hautgefühl | weich, glatt, samtig | weich, glatt, samtig | weich, glatt, samtig | weich, glatt, etwas ölig | weich, glatt, samtig | trocken, etwas klebrig | trocken, klebrig |

### Weitere Emulsionsbeispiele:

Diese Beispiele sollen zeigen, dass die erfindungsgemäßen Emulgatoren in einer Vielzahl kosmetischer Formulierungen eingesetzt werden können. Je nach Hydrophilie der erfindungsgemäßen Emulgatoren können O/W oder W/O Emulsionen hergestellt werden. Es ist darüber hinaus mit Hilfe der erfindungsgemäßen Emulgatoren möglich, Pigmente oder Festkörper stabil in Emulsionspräparate einzuarbeiten. Weiterhin zeigen die Beispiele die gute Kompatibilität mit typischen Co-Emulgatoren, Ölen, Verdickern und Stabilisatoren.

### O/W Emulsionsbeispiele

**Anti-Aging Tagescreme**

| Beispiel | **5** |
|---|---|
| Emulgator 1 | 1,50% |
| Ceteareth-25 | 1,00% |
| Stearyl Alcohol | 1,50% |
| Glyceryl Stearate | 3,00% |
| Stearic Acid | 1,50% |
| Myristyl Myristate | 1,00% |
| Ceramide IIIB | 0,10% |
| Caprylic/Capric Triglyceride | 5,00% |
| Ethylhexyl Palmitate | 4,40% |
| Ethylhexyl Methoxycinnamate | 2,00% |
| Butyl Methoxydibenzoylmethane | 1,00% |
| Glycerin | 3,00% |
| Water | ad 100% |
| TEGO® Carbomer 134 (Carbomer) | 0,10% |
| Ethylhexyl Palmitate | 0,40% |
| Sodium Hydroxide (10% in water) | q.s. |
| Preservative | q.s. |
| Parfum | q.s. |

**Selbstbräunende Köperlotion:**

| Beispiel | **6** |
|---|---|
| Emulgator 2 | 1,00% |
| ABIL® Care 85 (Bis-PEG/PPG-16/16 PEG/PPG-16/16 Dimethicone; Caprylic/Capric Triglyceride) | 1,00% |
| Cetearyl Isononanoate | 5,00% |
| Decyl Cocoate | 5,00% |
| Isopropyl Myristate | 5,00% |
| Sepigel® 305 (Polyacrylamide; C13-14 Isoparaffin; Laureth-7) | 1,50% |
| PEG-30 Glyceryl Stearate | 2,00% |
| Dihydroxyacetone | 5,00% |
| Propylene Glycol | 3,00% |
| Water | ad 100% |
| Citric Acid | q.s. |
| Preservative | q.s. |
| Parfum | q.s. |

**Kationische Sonnenschutzcreme (in-vitro SPF 18):**

| Beispiel | **7** |
|---|---|
| Emulgator 1 | 2,00% |
| Distearyldimonium Chloride | 1,50% |
| Glyceryl Stearate | 2,00% |
| Stearyl Alcohol | 1,00% |
| C12-15 Alkyl Benzoate | 5,00% |
| TEGO® Sun TDEC 45 (Titanium Dioxide; Diethylhexyl Carbonate; Polyglyceryl-6 Polyhydroxystearate)) | 5,00% |
| Diethylhexyl Carbonate | 3,50% |
| Cetyl Ricinoleate | 1,00% |
| Triisostearin | 1,00% |
| Octocrylene | 3,00% |
| Ethylhexyl Methoxycinnamate | 4,00% |
| Butyl Methoxydibenzoylmethane | 2,00% |
| Water | ad 100% |
| Glycerin | 3,00% |
| Preservative | q.s. |
| Parfum | q.s. |

**Hautglättende Bodylotion:**

| Beispiel | **8** |
|---|---|
| Emulgator 1 | 1,00% |
| ABIL® Care 85 (Bis-PEG/PPG-16/16 PEG/PPG-16/16 Dimethicone; Caprylic/Capric Triglyceride) | 1,00% |
| Diethylhexyl Carbonate | 7,00% |
| Isopropyl Palmitate | 7,60% |
| Polysorbate 20 | 0,20% |
| Creatine | 0,50% |
| Panthenol | 0,50% |
| Glycerin | 3,00% |
| Water | ad 100% |
| TEGO® Carbomer 341 ER (Acrylates / C10-30 Alkyl Acrylate Crosspolymer) | 0,30% |
| Xanthan Gum | 0,10% |
| Sodium Hydroxide (10% in water) | q.s. |
| TEGO® Smooth Complex (Betaine; Urea; Potassium Lactate; Polyglutamic Acid; Hydrolyzed Sclerotium Gum) | 2,00% |
| Preservative | q.s. |
| Parfum | q.s. |

**Seidiges Cremegel:**

| Beispiel | **9** |
|---|---|
| Emulgator 2 | 2,00% |
| Bis-PEG/PPG-14/14 Dimethicone | 2,00% |
| Cyclomethicone | 10,00% |
| Dimethicone | 3,00% |
| Cetyl Ricinoleate | 2,00% |
| Xanthan Gum | 0,20% |
| TEGO® Carbomer 341 ER (Acrylates / C10-30 Alkyl Acrylate Crosspolymer) | 0,40% |
| Caprylic/Capric Triglyceride | 1,90% |
| Water | ad 100% |
| PEG/PPG-20/20 Dimethicone | 1,00% |
| Alcohol | 5,00% |
| Sodium Hydroxide (10% in water) | q.s. |
| Preservative | q.s. |
| Parfum | q.s. |

### W/O Emulsionsbeispiele

**W/Si Lotion**

| Beispiel | **10** |
|---|---|
| Emulgator 3 | 1,50% |
| Cyclopentasiloxane | 19,50% |
| NaCl | 0,50% |
| Glycerin | 3,00% |
| Water | ad 100% |
| Preservative | q.s. |
| Parfum | q.s. |

**Wasserfester Sonnenschutz:**

| Beispiel | **11** |
|---|---|
| Emulgator 3 | 2,50% |
| C12-15 Alkyl Benzoate | 10,00% |
| Paraffinum Perliquidum | 13,50% |
| Cetyl Dimethicone | 1,00% |
| Titanium Dioxide | 5,00% |
| Sodium Chloride | 0,50% |
| Water | ad 100% |
| Preservative | q.s. |
| Parfum | q.s. |

**Make-Up Foundation:**

| Beispiel | **12** |
|---|---|
| Emulgator 3 | 3,00% |
| Diethylhexyl Carbonate | 10,00% |
| Cyclopentasiloxane | 12,60% |
| Iron Oxides | 1,80% |
| Titanium Dioxide | 7,20% |
| Talc | 2,00% |
| Ethylhexyl Palmitate | 3,40% |
| NaCl | 1,00% |
| Glycerin | 2,00% |
| Water | ad 100% |
| Preservative | q.s. |
| Parfum | q.s. |

**O/W Tränkemulsion für kosmetische Feuchttücher**

| | Beispiel | **13** |
|---|---|---|
| A | TEGO® Wipe DE (Diethylhexyl Carbonate; Polyglyceryl-4 Laurate; Phenoxyethanol; Methyl-paraben; Dilauryl Citrate; Ethylparaben; Butyl-paraben; Propylparaben; Isobutylparaben) | 5,70% |
| B | Demineralized water | 5,70% |
| C | Emulgator 1 | 0,30% |
| | Creatine | 0,25% |
| | Panthenol | 0,50% |
| | Demineralized water | 93,25 |
| Z | Parfum | q.s. |

| | | |
|---|---|---|
| Herstellung: Bei Raumtemperatur wird zunächst A mit B gemischt, dann werden unter Rühren C und Z zugegeben. | | |

## Patentansprüche

1. Verwendung mindestens eines Emulgator-Systems, enthaltend organomodifizierte Siloxanblockcopolymere, erhältlich durch
A) Anlagerung von Organopolysiloxanen der allgemeinen Formel I 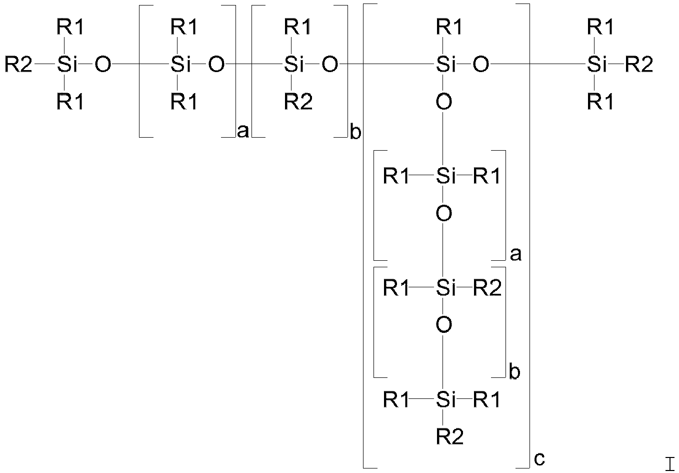 worin
R¹ gleiche oder verschiedene aliphatische oder aromatische Kohlenwasserstoffreste mit 1 bis 20 C-Atomen,
R² R¹ oder H, mit der Maßgabe, dass mindestens drei Reste R² gleich H sind,
a 5 bis 500, b 1 bis 50,
c 0 bis 5
bedeuten,
an Doppelbindungen enthaltende Siloxane der allgemeinen Formel II 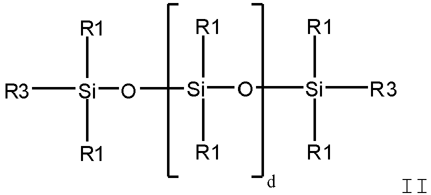 mit
d 10 bis 1.000 und R³ unabhängig voneinander gleiche oder verschiedene, mindestens eine Doppelbindung enthaltende Kohlenwasserstoffreste mit 2 bis 12 C-Atomen,
in Gegenwart von Platin- oder Rhodiumkatalysatoren, mit der Maßgabe, dass die Organopolysiloxane der allgemeinen Formel I im mindestens 6-fachen molaren Überschuss, bezogen auf das Doppelbindungen enthaltende Siloxan der allgemeinen Formel II, vorliegt, unter Erhalt eines Si-H-Gruppen aufweisenden Reaktionsproduktes und weiterer Umsetzung des Reaktionsproduktes in mindestens einer der Stufen
B) übergangsmetall-katalysierter teilweiser oder vollständiger Addition der SiH-Gruppen an Alkenyl- und/oder Alkinylverbindungen,
oder
C) teilweise oder vollständige Umsetzung der nach obiger Reaktion(en) verbliebenen Si-H-Gruppen in Gegenwart eines Katalysators mit mindestens einem Alkohol, aus der Gruppe der linearen oder verzweigten, gesättigten, ein- oder mehrfach ungesättigten, aromatischen, aliphatisch-aromatischen, gegebenenfalls Halogenatome enthaltenden Monoalkohole, Polyethermonoalkohole, Polyestermonoalkohole, Aminoalkohole.
als Emulgator oder als Dispergierhilfsmittel in kosmetischen, dermatologischen oder pharmazeutischen Formulierungen.

2. Verwendung gemäß Anspruch 1, **dadurch gekennzeichnet, dass** R¹ gleiche oder verschiedene unverzweigte, aliphatische oder aromatische Kohlenwasserstoffreste mit 1 bis 9 C-Atomen sind.

3. Verwendung gemäß mindestens einem der Ansprüche 1 oder 2, **dadurch gekennzeichnet, dass** im Schritt B) an Doppelbindungen enthaltende Polyether addiert wird.

4. Verwendung gemäß mindestens einem der Ansprüche 1 bis 3, **dadurch gekennzeichnet, dass** im Schritt B) an Allylpolyether addiert wird.

5. Verwendung gemäß mindestens einem der Ansprüche 1 bis 4, **dadurch gekennzeichnet, dass** als Katalysator in Stufe C) borhaltige Lewissäuren eingesetzt werden.

6. Verwendung gemäß Anspruch 5, **dadurch gekennzeichnet, dass** als borhaltige Lewissäure Tris(pentafluorphenyl)boran (C₆F₅)₃B eingesetzt wird.

7. Verwendung gemäß mindestens einem der Ansprüche 1 bis 6, **dadurch gekennzeichnet, dass** als Katalysator in Schritt C) eine katalytische Mischung enthaltend mindestens eine Carbonsäure und mindestens ein Salz einer Carbonsäure eingesetzt wird.

8. Kosmetische Formulierung oder Pflege- und Reinigungsmittel enthaltend mindestens eines der Emulgator-Systeme wie in einem der Ansprüche 1 bis 7 genannt, und als zusätzliche Komponente enthaltend Partikel oder Pigmente.

9. Kosmetische Formulierung oder Pflege- und Reinigungsmittel enthaltend mindestens eines der Emulgator-Systeme wie in einem der Ansprüche 1 bis 7 genannt, und als zusätzliche Komponente enthaltend kosmetische Wirkstoffe.

## Claims

1. Use of at least one emulsifier system comprising organomodified siloxane block copolymers obtainable by
A) addition reaction of organopolysiloxanes of the general formula I 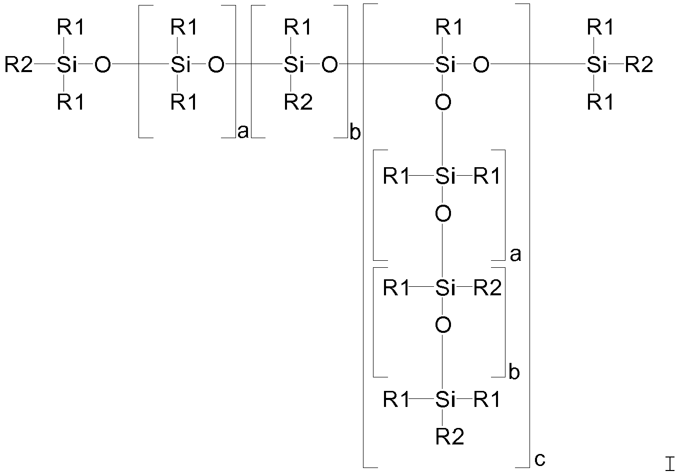 in which
R¹ are identical or different aliphatic or aromatic hydrocarbon radicals having 1 to 20 carbon atoms,
R² is R¹ or H, with the proviso that at least three radicals R² are H,
a is 5 to 500,
b is 1 to 50,
c is 0 to 5,
onto siloxanes of the general formula II containing double bonds 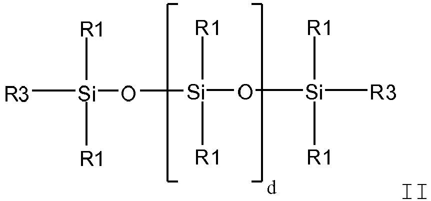 where
d is 10 to 1000 and R³, independently of one another, are identical or different hydrocarbon radicals having 2 to 12 carbon atoms and containing at least one double bond,
in the presence of platinum or rhodium catalysts, with the proviso that the organopolysiloxanes of the general formula I are present in at least 6-fold molar excess, based on the siloxane of the general formula II containing double bonds, to give a reaction product having Si-H groups and with further reaction of the reaction product in at least one of the stages
B) transition-metal-catalysed partial or complete addition of the SiH groups onto alkenyl and/or alkynyl compounds,
or
C) partial or complete reaction of the Si-H groups remaining after the above reaction(s) in the presence of a catalyst with at least one alcohol, from the group of linear or branched, saturated, mono- or polyunsaturated, aromatic, aliphatic-aromatic, optionally halogen-atom-containing monoalcohols, polyether monoalcohols, polyester monoalcohols, amino alcohols, as emulsifier or as dispersion auxiliary in cosmetic, dermatological or pharmaceutical formulations.

2. Use according to Claim 1, **characterized in that** R¹ are identical or different unbranched, aliphatic or aromatic hydrocarbon radicals having 1 to 9 carbon atoms.

3. Use according to at least one of Claims 1 or 2, **characterized in that**, in step B), the addition is onto polyethers containing double bonds.

4. Use according to at least one of Claims 1 to 3, **characterized in that**, in step B) the addition is onto allyl polyether.

5. Use according to at least one of Claims 1 to 4, **characterized in that** boron-containing Lewis acids are used as catalyst in stage C).

6. Use according to Claim 5, **characterized in that** tris(pentafluorophenyl)borane (C₅F₄)3B is used as boron-containing Lewis acid.

7. Use according to at least one of Claims 1 to 6, **characterized in that** a catalytic mixture comprising at least one carboxylic acid and at least one salt of a carboxylic acid is used as catalyst in step C).

8. Cosmetic formulation or care and cleaning composition comprising at least one of the emulsifier systems as mentioned in one of Claims 1 to 7, and comprising particles or pigments as additional component.

9. Cosmetic formulation or care and cleaning composition comprising at least one of the emulsifier systems as mentioned in one of Claims 1 to 7, and comprising cosmetic active ingredients as additional component.

## Revendications

1. Utilisation d'au moins un système émulsifiant, contenant des copolymères séquencés de siloxane organo-modifiés, pouvant être obtenus par
A) l'addition d'organopolysiloxanes de formule générale I 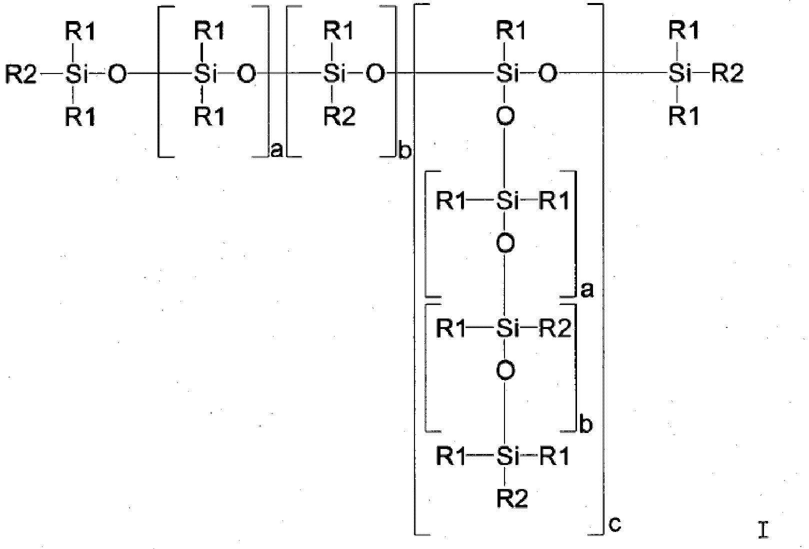 dans laquelle
les R¹ sont des radicaux hydrocarbonés aliphatiques ou aromatiques identiques ou différents de 1 à 20 atomes C, les R² représentent R¹ ou H, à condition qu'au moins trois radicaux R² représentent H,
a signifie 5 à 500,
b signifie 1 à 50,
c signifie 0 à 5,
sur des siloxanes contenant des doubles liaisons de formule générale II 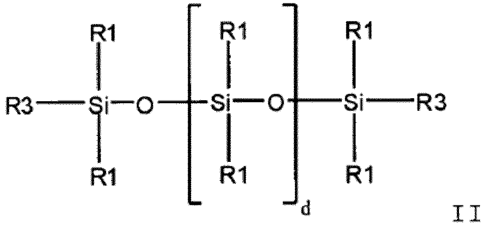 dans laquelle
d signifie 10 à 1 000, et les R³ représentent indépendamment les uns des autres des radicaux hydrocarbonés identiques ou différents, contenant au moins une double liaison, de 2 à 12 atomes C,
en présence de catalyseurs de platine ou de rhodium, à condition que les organopolysiloxanes de formule générale I soient présents en un excès molaire d'un facteur d'au moins 6 par rapport au siloxane contenant des doubles liaisons de formule générale II, pour obtenir un produit de réaction comprenant des groupes Si-H, et la mise en réaction ultérieure du produit de réaction dans au moins une des étapes suivantes :
B) l'addition partielle ou totale catalysée par un métal de transition des groupes SiH sur des composés d'alcényle et/ou d'alcynyle,
ou
C) la mise en réaction partielle ou totale des groupes Si-H restant après la ou les réactions précédentes en présence d'un catalyseur avec au moins un alcool du groupe constitué par les monoalcools linéaires ou ramifiés, saturés, mono- ou polyinsaturés, aromatiques, aliphatiques-aromatiques, contenant éventuellement des atomes d'halogène, les polyéther-monoalcools, les polyester-monoalcools, les aminoalcools,
en tant qu'émulsifiant ou en tant qu'adjuvant de dispersion dans des formulations cosmétiques, dermatologiques ou pharmaceutiques.

2. Utilisation selon la revendication 1, **caractérisée en ce que** les R¹ sont des radicaux hydrocarbonés non ramifiés, aliphatiques ou aromatiques, identiques ou différents, de 1 à 9 atomes C.

3. Utilisation selon au moins l'une quelconque des revendications 1 ou 2, **caractérisée en ce qu'**à l'étape B), l'addition est effectuée sur des polyéthers contenant des doubles liaisons.

4. Utilisation selon au moins l'une quelconque des revendications 1 à 3, **caractérisée en ce qu'**à l'étape B), l'addition est effectuée sur des polyéthers allyliques.

5. Utilisation selon au moins l'une quelconque des revendications 1 à 4, **caractérisée en ce que** des acides de Lewis contenant du bore sont utilisés en tant que catalyseur à l'étape C).

6. Utilisation selon la revendication 5, **caractérisée en ce que** du tris (pentafluorophényl) borane (C₆F₅)₃B est utilisé en tant qu'acide de Lewis contenant du bore.

7. Utilisation selon au moins l'une quelconque des revendications 1 à 6, **caractérisée en ce qu'**un mélange catalytique contenant au moins un acide carboxylique et au moins un sel d'un acide carboxylique est utilisé en tant que catalyseur à l'étape C).

8. Formulation cosmétique ou agent de soin et de nettoyage contenant au moins un des systèmes émulsifiants tels qu'indiqués dans l'une quelconque des revendications 1 à 7, et contenant des particules ou des pigments en tant que composant supplémentaire.

9. Formulation cosmétique ou agent de soin et de nettoyage contenant au moins un des systèmes émulsifiants tels qu'indiqués dans l'une quelconque des revendications 1 à 7, et contenant des agents actifs cosmétiques en tant que composant supplémentaire.
